# EUROPÄISCHE PATENTANMELDUNG

(11) **EP 3 682 918 A1**
(43) Veröffentlichungstag der Anmeldung: **22.07.2020**
(21) Anmeldenummer: 19152910.6
(22) Anmeldetag: 21.01.2019
(51) Int. Cl.: A61M 1/10

(54) **FLUIDPUMPE**

(71) Anmelder: Berlin Heart GmbH, 12247 Berlin (DE)
(72) Erfinder: Wisniewski, Adrian, 10963 Berlin (DE); Müller, Jörg, 10439 Berlin (DE); Schmidt, Bodo, 14513 Teltow (DE)
(74) Vertreter: Pfenning, Meinig & Partner mbB

(57) **Zusammenfassung**

Die vorliegende Erfindung betrifft Fluidpumpe zum Fördern eines Fluids, insbesondere von Blut, umfassend: ein Gehäuse mit einem Fluideinlass und einem Fluidauslass und einen Rotor, welcher innerhalb des Gehäuses zur Förderung des Fluids vom Fluideinlass zum Fluidauslass um eine Rotationsachse drehbar angeordnet ist, wobei der Rotor in dem Gehäuse mittels eines mechanischen Lagers gelagert ist. Die erfindungsgemäße Fluidpumpe zeichnet sich dadurch aus, dass ein Verlauf eines Strömungsquerschnitts zwischen dem Rotor und dem Gehäuse in Richtung der Rotationsachse im Bereich des mechanischen Lagers ein lokales oder bereichsweises Strömungsquerschnittsminimum aufweist.

## Beschreibung

Die vorliegende Erfindung betrifft eine Fluidpumpe zum Fördern eines Fluids, insbesondere zum Fördern von Blut.

Ein bekanntes Problem von intrakorporalen Blutpumpen mit mechanischen Lagern ist eine unzureichende Lagerauswaschung und ein unzureichender Abtrag von im Lager entstehender Wärme, welche beispielsweise durch Reibung der Lagerelemente entsteht. Eine unzureichende Lagerauswaschung und ein unzureichender Wärmeabtrag in der Blutpumpe können das Risiko für eine Thrombenbildung und eine Blutgerinnung innerhalb der Blutpumpe erhöhen und damit eine Lebensgefahr für den Patienten darstellen.

Eine Aufgabe der vorliegenden Erfindung ist es daher, eine Fluidpumpe bereitzustellen, welche eine verbesserte Lagerauswaschung und einen größeren Wärmeabtrag im Bereich des Lagers ermöglicht.

Die Aufgabe wird durch eine Fluidpumpe gemäß Anspruch 1 gelöst. Vorteilhafte Weiterbildungen der erfindungsgemäßen Fluidpumpe sind in den abhängigen Ansprüchen 2 bis 13 aufgeführt.

Die erfindungsgemäße Fluidpumpe zum Fördern eines Fluids, insbesondere von Blut, umfasst ein Gehäuse mit einem Fluideinlass und einem Fluidauslass und einen Rotor, welcher innerhalb des Gehäuses zur Förderung des Fluids vom Fluideinlass zum Fluidauslass um eine Rotationsachse drehbar angeordnet ist. Der Rotor ist in dem Gehäuse mittels eines mechanischen Lagers gelagert. Die erfindungsgemäße Fluidpumpe zeichnet sich dadurch aus, dass ein Verlauf eines Strömungsquerschnitts zwischen dem Rotor und dem Gehäuse in Richtung der Rotationsachse im Bereich des mechanischen Lagers ein lokales oder bereichsweises Strömungsquerschnittsminimum aufweist.

Unter dem Strömungsquerschnitt wird hier eine effektive Strömungsquerschnittsfläche innerhalb der Blutpumpe radial zur Rotationsachse verstanden, durch welche das geförderte Fluid zwischen dem Lager bzw. dem Rotor und dem Gehäuse hindurchfließt.

Weiterhin wird unter einem lokalen Strömungsquerschnittsminimum des Verlaufs des Strömungsquerschnitts in Richtung der Rotationsachse im Bereich des mechanischen Lagers eine lokale Verringerung des Strömungsquerschnitts bzw. der Strömungsquerschnittsfläche auf gleicher axialer Höhe wie das mechanische Lager, bezogen auf die Rotationsachse, verstanden.

Weiterhin wird unter einem bereichsweisen Strömungsquerschnittsminimum des Verlaufs des Strömungsquerschnitts in Richtung der Rotationsachse im Bereich des mechanischen Lagers eine Verringerung der Strömungsquerschnittsfläche verstanden, welche auf gleicher axialer Höhe wie das mechanische Lager, bezogen auf die Rotationsachse, einen Minimalwert erreicht und über einen bestimmten Bereich ihres Verlaufs in Richtung der Rotationsachse auf diesem Minimalwert bleibt.

Unter dem Bereich des mechanischen Lagers oder Lagerbereich wird im Wesentlichen ein Strömungsbereich des Fluids verstanden, welcher sich radial zum und entlang des ersten und zweiten Lagerelements, also radial zum und entlang des gesamten mechanischen Lagers erstreckt.

Im Folgenden bezieht sich die Formulierung "axiale Höhe" immer auf die Rotationsachse. Auf "gleicher axialer Höhe" bedeutet dabei auch "radial zu".

Durch die Bildung eines Strömungsquerschnittsminimums im Bereich des mechanischen Lagers kann der geförderte Fluidstrom im Bereich des mechanischen Lagers beschleunigt werden. Durch die Beschleunigung des Fluidstroms wird das mechanische Lager besser ausgewaschen und im Lager entstehende Wärme, beispielsweise durch Reibung der Lagerelemente, kann schneller abtransportiert werden. Hierdurch verringert sich bei einer Blutpumpe das Risiko der Thrombenbildung und Blutgerinnung und somit eine Gefahr für den Patienten.

Ein größerer Wärmeabtrag kann ferner durch eine Erhöhung der Wandschubspannung und damit des Wärmeübergangs, durch eine verbesserte Durchmischung des Fluids im Bereich des Lagers oder durch eine verbesserte Ableitung der Wärme in den sich in der Fluidpumpe befindlichen Festkörpern durch eine Wahl von entsprechenden Materialien erreicht werden.

Das mechanische Lager kann insbesondere eine erste mit dem Gehäuse verbundene Lagerkomponente und eine zweite mit dem Rotor verbundene Lagerkomponente aufweisen, wobei die erste und die zweite Lagerkomponente in Richtung der Rotationsachse in einem Kontaktbereich bis auf einen Lagerspalt aneinander anliegen, wobei sich das lokale oder bereichsweise Strömungsquerschnittsminimum im Bereich der ersten Lagerkomponente, insbesondere auf gleicher axialer Höhe wie die erste Lagerkomponente, im Bereich der zweiten Lagerkomponente, insbesondere auf gleicher axialer Höhe wie die zweite Lagerkomponente, und/oder im Bereich des Kontaktbereichs, insbesondere auf gleicher axialer Höhe wie der Kontaktbereich, befindet.

Weiterhin kann das Gehäuse eine einen Fluidbereich begrenzende und in einer Umfangsrichtung um die Rotationsachse geschlossene innere Wandung aufweisen, und das lokale oder bereichsweise Strömungsquerschnittsminimum daraus resultieren, dass ein Verlauf eines Wandungsdurchmessers der inneren Wandung in Richtung der Rotationsachse im Bereich des mechanischen Lagers ein lokales oder bereichsweises Minimum aufweist.

Insbesondere kann der Verlauf des Wandungsdurchmessers im Bereich der ersten Lagerkomponente, im Bereich der zweiten Lagerkomponente und/oder im Bereich des Kontaktbereichs ein lokales oder bereichsweises Minimum aufweisen.

Weiterhin kann das lokale Strömungsquerschnittsminimum daraus resultieren, dass ein Verlauf eines Lagerdurchmessers des mechanischen Lagers in Richtung der Rotationsachse ein lokales oder bereichsweises Maximum aufweist. Insbesondere kann der Verlauf des Lagerdurchmessers im Bereich der ersten Lagerkomponente, im Bereich der zweiten Lagerkomponente und/oder im Bereich des Kontaktbereichs ein lokales oder bereichsweises Maximum aufweisen. Besonders vorteilhaft ist es, wenn die zweite Lagerkomponente einen größeren Durchmesser aufweist als die erste Lagerkomponente, die zweite Lagerkomponente also in Bezug auf die erste Lagerkomponente aufgeweitet ist.

Der Strömungsquerschnitt kann im lokalen oder bereichsweisen Strömungsquerschnittsminimum um ≥ 10% und/oder ≤ 50%, insbesondere ≥ 20% und/oder ≤ 40%, vorzugsweise 30% verglichen mit einem Strömungsquerschnitt in einem dem Lagerbereich stromaufwärts und/oder stromabwärts benachbarten Bereich verringert sein.

Weiterhin kann die Fluidpumpe derart ausgebildet sein, dass sich der Strömungsquerschnitt in Richtung der Rotationsachse stromaufwärts des Strömungsquerschnittsminimums um ≥ 2 % des Strömungsquerschnittsminimums pro mm und/oder ≤ 30 % des Strömungsquerschnittsminimums pro mm und stromabwärts des Strömungsquerschnittsminimums um > 0 % des Strömungsquerschnittsminimums pro mm und/oder ≤ 20 % des Strömungsquerschnittsminimums pro mm, insbesondere ≤ 10 % des Strömungsquerschnittsminimums pro mm vergrößert. Unter der Vergrößerung des Strömungsquerschnitts stromaufwärts des Strömungsquerschnittsminimums wird eine Verkleinerung des Strömungsquerschnitts in Stromrichtung des Fluids vor dem und bis zum Strömungsquerschnittsminimum verstanden. Eine langsame oder nur geringe Vergrößerung des Strömungsquerschnitts stromabwärts, also in Strömungsrichtung des Fluids, weist ein geringeres Ablöse- und Verwirbelungsrisiko der Fluidströmung, also des stromabwärts strömenden Fluids, und damit im Falle von Blut ein geringeres Risiko einer Thrombenbildung in der Fluidpumpe auf. Eine schnellere oder stärkere Vergrößerung des Strömungsquerschnitts stromaufwärts stellt gleichzeitig eine schnellere oder stärkere Verkleinerung des Strömungsquerschnitts in Strömungsrichtung dar und ermöglicht so eine größere Beschleunigung des durch die Fluidpumpe strömenden Fluids, also eine größere Beschleunigung des Fluids stromabwärts, und somit eine schnellere Auswaschung und einen schnelleren Wärmeabtrag.

Ferner kann eine maximale Vergrößerung des Strömungsquerschnitts in Richtung der Rotationsachse stromaufwärts des lokalen oder bereichsweisen Strömungsquerschnittsminimums größer sein als oder gleich groß sein wie stromabwärts des lokalen oder bereichsweisen Strömungsquerschnittsminimums. Ist die Vergrößerung stromaufwärts und stromabwärts gleich groß, ist das Strömungsquerschnittsminimum symmetrisch. Ein derartiges Strömungsquerschnittsminimum kann durch ein symmetrisches Minimum des Wandungsdurchmessers und/oder durch ein symmetrisches Maximum des Lagerdurchmessers realisiert sein. Ein symmetrisches Strömungsquerschnittsminimum ist einfach und auch über einen kleinen Bereich des Verlaufs des Strömungsquerschnitts zu realisieren und damit insbesondere für kleine Fluidpumpen vorteilhaft. Ist die Vergrößerung stromaufwärts größer als stromabwärts kann der Fluidstrom zur schnelleren Auswaschung und Kühlung sowohl schnell beschleunigt als auch das Ablöserisiko des Fluidstroms minimiert werden.

Das lokale oder bereichsweise Strömungsquerschnittsminimum kann ein bereichsweises Strömungsquerschnittsminimum sein und sich vorzugsweise im Bereich des Kontaktbereichs sowie stromabwärts des Kontaktbereichs, insbesondere im Bereich des zweiten Lagerelements befinden. Ein derartiges bereichsweises Strömungsquerschnittsminimum weist ähnliche Vorteile auf, wie das oben beschriebene Strömungsquerschnittsminimum mit größerer Steigung stromaufwärts als stromabwärts, nämlich eine beschleunigte Auswaschung und Kühlung bei geringem Ablöserisiko des Fluidstroms.

Das lokale oder bereichsweise Strömungsquerschnittsminimum kann insbesondere derart ausgebildet sein, dass eine Wandschubspannung im Bereich des mechanischen Lagers ein lokales Maximum aufweist oder maximal ist.

Alternativ oder zusätzlich zur Ausbildung eines Strömungsquerschnittsminimums im Bereich des mechanischen Lagers und den vorstehend beschriebenen Ausführungsbeispielen kann eine Verbesserung des Wärmeabtrags auch durch Optimierung der Lagergeometrie, insbesondere der Geometrie der einzelnen Lagerkomponenten erreicht werden. Hierbei kann eine Oberfläche des Lagers vergrößert werden, insbesondere indem der Lagerdurchmesser erhöht wird, das Lager in Richtung der Rotationsachse länger gestaltet wird und/oder eine Oberfläche der ersten und zweiten Lagerkomponente eine sonstige oberflächenvergrößernde Geometrie aufweist. Insbesondere ist es vorteilhaft, wenn das Lager eine Länge von ≥ 3mm, insbesondere 5 bis 8 mm aufweist, um eine Verbesserung des Wärmeabtrags zu ermöglichen.

Alternativ oder zusätzlich zur Ausbildung eines Strömungsquerschnittsminimums im Bereich des mechanischen Lagers und den vorstehend beschriebenen Ausführungsbeispielen kann eine Verbesserung des Wärmeabtrags oder eine Verringerung der Wärmeentstehung durch Reibung zwischen den Lagerelementen auch durch eine Optimierung einer Materialauswahl für das mechanische Lager erreicht werden. Besonders vorteilhaft ist es, wenn das erste und/oder das zweite Lagerelement ein Material mit einem geringen Reibkoeffizienten und/oder mit einem hohen Wärmeleitungskoeffizienten, insbesondere Keramik, Sintermetall und/oder Diamant aufweist oder daraus besteht. Weiterhin kann das erste und/oder zweite Lagerelement mit einem Material mit geringem Reibkoeffizienten und/oder hohem Wärmeleitungskoeffizienten, insbesondere mit Keramik, Sintermetall und/oder Diamant beschichtet sein.

Eine weitere Möglichkeit zur Optimierung der Auswaschung des Lagerbereichs des mechanischen Lagers besteht darin alternativ oder zusätzlich zur Ausbildung eines Strömungsquerschnittsminimums im Bereich des mechanischen Lagers und den vorstehend beschriebenen Ausführungsbeispielen stromaufwärts des mechanischen Lagers ein Vorlaufrad anzuordnen zur Erzeugung von entsprechend gerichteten Wirbeln in der Fluidströmung, welche die Auswaschung des mechanischen Lagers verbessern.

Weiterhin ist es denkbar zusätzlich oder alternativ zu den vorstehend beschriebenen Lösungen, das erste oder zweite Lagerelement mit Drehelementen oder Schaufeln am Rotor auszustatten, welche stromaufwärts über das mechanische Lager ragen. Diese Drehelemente können insbesondere zur Erzeugung von Wirbeln und Turbulenz im Lagerbereich ausgebildet sein, sodass eine Auswaschung und Kühlung des Lagerbereichs des mechanischen Lagers verbessert wird.

Weiterhin ist es denkbar zusätzlich oder alternativ zu den vorstehend beschriebenen Lösungen, stromaufwärts des mechanischen Lagers entsprechend ausgebildete und mit dem Gehäuse der Fluidpumpe verbundene Streben als Strömungselemente vorzusehen, welche die Fluidströmung und/oder die Wandschubspannung im Lagerbereich erhöhen und so eine Auswaschung des Lagerbereichs des mechanischen Lagers und einen Wärmeabtrag verbessern.

Weiterhin ist es denkbar zusätzlich oder alternativ zu den vorstehend beschriebenen Lösungen den Rotor mit einer Unwucht auszustatten, welche derart ausgebildet ist, dass die Auswaschung im Bereich des mechanischen Lagers verbessert wird.

Weiterhin ist es denkbar zusätzlich oder alternativ zu den vorstehend beschriebenen Lösungen eine Oberfläche des ersten und/oder zweiten Lagerelements mit einer Struktur oder Texturierung zu versehen, insbesondere eine Vielzahl von Vertiefungen zur Erzeugung eines Golfball-Effekts auf der Oberfläche des ersten und/oder zweiten Lagerelements anzuordnen. Hierdurch wird die Fluidströmung derart manipuliert, dass eine Lagerauswaschung und Kühlung des mechanischen Lagers verbessert wird.

Weiterhin ist es denkbar zusätzlich oder alternativ zu den vorstehend beschriebenen Lösungen stromaufwärts des mechanischen Lagers eine Geometrie anzuordnen, welche ausgebildet ist, Taylorwirbel zur Verbesserung der Lagerauswaschung und Kühlung sowie Erhöhung der Wandschubspannung im Bereich des mechanischen Lagers zu induzieren.

Im Folgenden wird eine erfindungsgemäße Fluidpumpe anhand von Figuren detaillierter beschrieben. Dabei werden verschiedene erfindungswesentliche oder auch vorteilhafte weiterbildende Elemente im Rahmen jeweils eines konkreten Beispiels genannt, wobei auch einzelne dieser Elemente als solche zur Weiterbildung der Erfindung - auch herausgelöst aus dem Kontext des jeweiligen Beispiels und weiterer Merkmale des jeweiligen Beispiels - verwendet werden können. Weiterhin werden in den Figuren für gleiche oder ähnliche Elemente gleiche oder ähnliche Bezugszeichen verwendet, und deren Erläuterung daher teilweise weggelassen.

Es zeigen
- Figur 1: eine schematische Längsschnittansicht einer erfindungsgemäßen Fluidpumpe gemäß einem ersten Ausführungsbeispiel,
- Figur 2: eine schematische Längsschnittansicht einer erfindungsgemäßen Fluidpumpe gemäß einem zweiten Ausführungsbeispiel,
- Figur 3: eine schematische Längsschnittansicht einer erfindungsgemäßen Fluidpumpe gemäß einem dritten Ausführungsbeispiel,
- Figur 4: eine schematische Längsschnittansicht einer erfindungsgemäßen Fluidpumpe gemäß einem vierten Ausführungsbeispiel und
- Figuren 5 bis 12: verschiedene Varianten möglicher Strömungsquerschnittsverläufe in schematischer Ansicht.

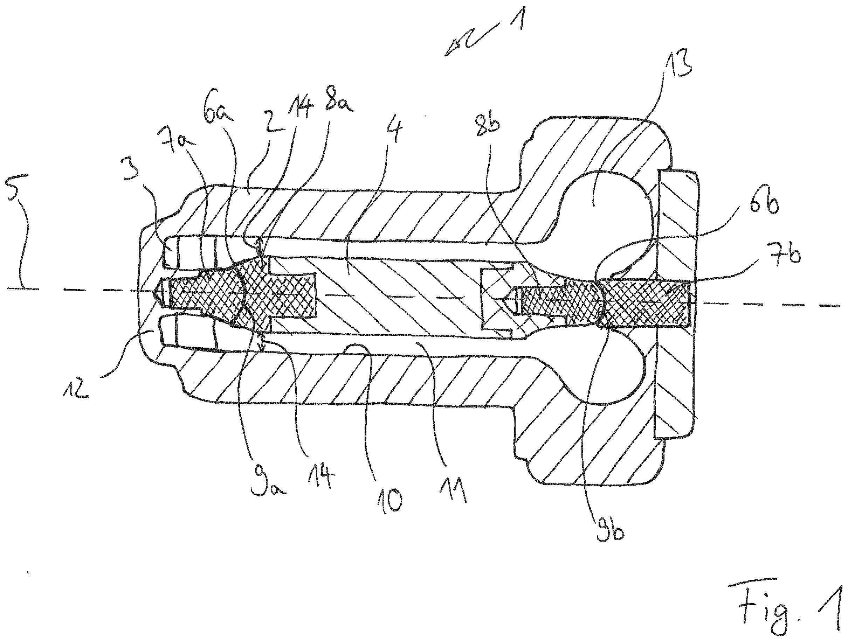

Figur 1 zeigt ein erstes Ausführungsbeispiel einer erfindungsgemäßen Fluidpumpe 1 in einer Längsschnittansicht. Bei der Fluidpumpe 1 handelt es sich um eine Rotationsfluidpumpe, welche ein Gehäuse 2 aufweist, das einen Stator der Rotationsfluidpumpe 1 bildet. Innerhalb des Gehäuses 2 ist ein Rotor 4 angeordnet, welcher sich um eine in Längsrichtung der Fluidpumpe 1 verlaufende Rotationsachse 5 dreht. Der Rotor 4 ist axial und radial mittels eines ersten mechanischen Lagers 6a und eines zweiten mechanischen Lagers 6b im Gehäuse 2 gelagert.

Das erste mechanische Lager 6a weist ein erstes Lagerelement 7a und ein zweites Lagerelement 8a auf. Das erste Lagerelement 7a ist über Streben 12, welche Teil einer Käfiggeometrie am Einlass der Fluidpumpe sind, starr mit dem Gehäuse 2 verbunden. Das zweite Lagerelement 8a ist starr mit dem Rotor 4 verbunden. In einem Kontaktbereich zwischen dem ersten Lagerelement 7a und dem zweiten Lagerelement 8a berühren sich das erste 7a und das zweite Lagerelement 8a bis auf einen Lagerspalt 9a. In dem Lagerspalt 9a kann sich ein Teil des von der Fluidpumpe geförderten Fluids befinden und einen Fluidfilm ausbilden, sodass das erste und das zweite Lagerelement 7a und 8a in dem Kontaktbereich aufeinander gleiten. Bei dem ersten mechanischen Lager 6a handelt es sich um ein Ball-Cup-Lager, wobei im Kontaktbereich das erste Lagerelement 7a eine konvexe sphärische Oberfläche aufweist und das zweite Lagerelement 8a eine konkave sphärische Oberfläche aufweist. Ebenso ist es denkbar, dass das erste Lagerelement 7a eine konkave sphärische Oberfläche aufweist und das zweite Lagerelement 8a eine konvexe sphärische Oberfläche aufweist. Der so sphärisch ausgebildete Kontaktbereich des ersten mechanischen Lagers ermöglicht sowohl eine Lagerung des Rotors 4 in axialer als auch in radialer Richtung.

Das zweite mechanische Lager 6b weist ebenso ein erstes Lagerelement 7b und eine zweites Lagerelement 8b auf, wobei das erste Lagerelement 7b starr im Gehäuse 2 verankert ist und das zweite Lagerelement 8b starr mit dem Rotor 4 verbunden ist. Zwischen dem ersten Lagerelement 7b und dem zweiten Lagerelement 8b berühren sich das erste 7b und das zweite Lagerelement 8b gegebenenfalls bis auf einen Lagerspalt 9b. In dem Lagerspalt kann sich ein Teil des von der Fluidpumpe geförderten Fluids befinden und einen Fluidfilm ausbilden, sodass das erste und das zweite Lagerelement 7b und 8b über den Fluidfilm aufeinander gleiten. Wie das erste mechanische Lager 6a ist auch das zweite mechanische Lager 6b als Ball-Cup-Lager mit einem sphärischen Kontaktbereich ausgebildet, wobei in dem Kontaktbereich eine Oberfläche des ersten Lagerelements 7b sphärisch konkav ausgebildet ist und eine Oberfläche des zweiten Lagerelements 8b sphärisch konvex ausgebildet ist. Der Kontaktbereich des zweiten mechanischen Lagers ermöglicht ebenfalls eine Lagerung des Rotors 4 sowohl in axialer als auch in radialer Richtung.

Die Fluidpumpe 1 weist einen Fluidbereich 11 zwischen dem Rotor 4 und einer dem Rotor 4 zugewandten inneren Wandung 10 des Gehäuses 2 auf, durch welchen das von der Fluidpumpe geförderte Fluid strömt. Das zu fördernde Fluid gelangt über einen im Bereich der Streben 12 angeordneten Fluideinlass 3 der Fluidpumpe in den Fluidbereich 11 und wird durch die Rotationsbewegung des Rotors 4 in Richtung der Rotationsachse 5 vom Fluideinlass 3 weg transportiert. Im Bereich des zweiten mechanischen Lagers 6b ist der Fluidbereich 11 zu einer Volute 13 verbreitert. In der Volute 13 geht die im Wesentlichen axiale Bewegung des Fluids in eine im Wesentlichen radiale Bewegung über, wobei das Fluid anschließend aus einem in der Volute 13 angeordneten Fluidauslass (hier nicht dargestellt) radial zur Rotationsachse 5 ausgestoßen wird.

Der Fluidbereich 11 weist einen sich in Richtung der Rotationsachse verändernden Strömungsquerschnitt auf. Im Bereich des ersten mechanischen Lagers 6a weist der Strömungsquerschnitt beim Übergang vom ersten Lagerelement 7a zum zweiten Lagerelement 7b in Strömungsrichtung eine Verkleinerung des Strömungsquerschnitts auf, wobei der Strömungsquerschnitt noch im Bereich des zweiten Lagerelements 8a ein Strömungsquerschnittsminimum 14 erreicht, welches sich in Strömungsrichtung über die übrige Länge des zweiten Lagerelements 8a sowie einen Großteil der Rotorlänge fortsetzt. Somit weist der Verlauf des Strömungsquerschnitts im Bereich des zweiten Lagerelements 8a ein bereichsweises Strömungsquerschnittsminimum 14 auf. Die Verkleinerung des Strömungsquerschnitts im Bereich des zweiten Lagerelements 8a wird durch eine graduelle Vergrößerung des Umfangs des zweiten Lagerelements 8a in Strömungsrichtung erreicht, wobei ein Wandungsdurchmesser der inneren Wandung im Bereich des ersten mechanischen Lagers 6a entlang der Rotationsachse 5 konstant ist. Der maximale Umfang des zweiten Lagerelements 8a ist dabei größer als der maximale Umfang des ersten Lagerelements 7a. Die Verkleinerung des Strömungsquerschnitts im Bereich des zweiten Lagerelements 8a ermöglicht eine Beschleunigung des Fluids über dem ersten mechanischen Lager 6a, wodurch dieses besser ausgewaschen und im Lager 6a entstehende Reibungswärme schneller abtransportiert wird.

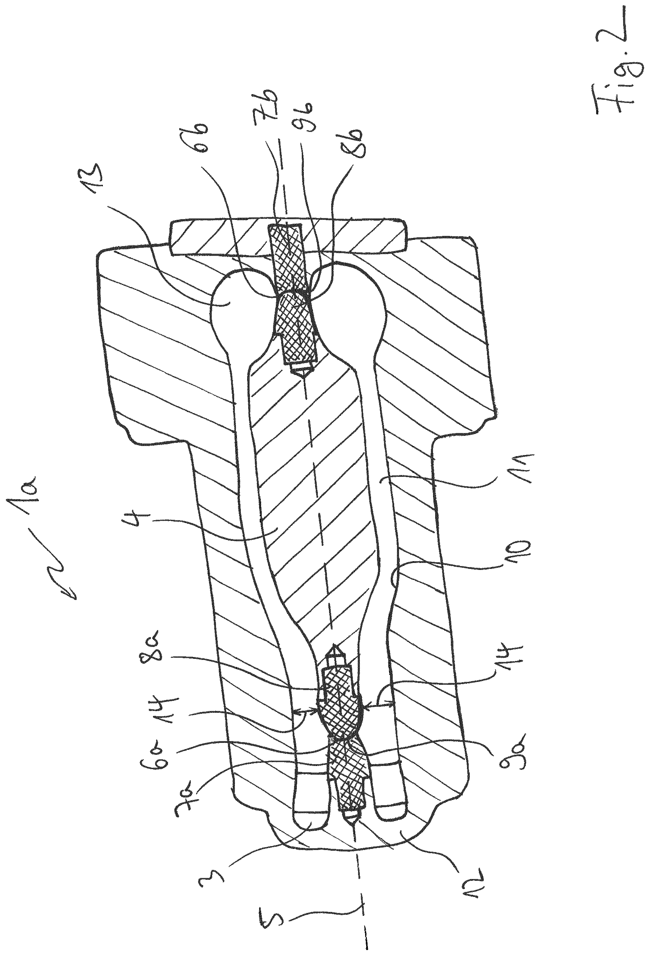

Figur 2 zeigt ein zweites Ausführungsbeispiel einer erfindungsgemäßen Fluidpumpe 1a in einer Längsschnittansicht. Die Fluidpumpe 1a ist ähnlich wie die Fluidpumpe 1 des ersten Ausführungsbeispiels ausgebildet. Die Fluidpumpe 1a unterscheidet sich von der Fluidpumpe 1 des ersten Ausführungsbeispiels im Wesentlichen lediglich in der Ausführung des ersten mechanischen Lagers 6a. Das erste mechanische Lager 6a der Fluidpumpe 1a ist ebenfalls als Ball-Cup-Lager ausgebildet, wobei jedoch das erste Lagerelement 7a im Kontaktbereich eine sphärisch konkave Oberfläche und das zweite Lagerelement 8a eine sphärisch konvexe Oberfläche aufweist, wobei das erste und das zweite Lagerelement 7a und 8a entlang ihrer einander zugewandten Oberflächen über einen sich im Lagerspalt 9a befindlichen Fluidfilm aufeinander gleiten können. Ähnlich wie im ersten Ausführungsbeispiel weist das zweite Lagerelement 8a in Strömungsrichtung eine graduelle Umfangsvergrößerung über einen maximalen Umfang des ersten Lagerelements 7a hinaus auf, welche bei einem im Bereich des ersten mechanischen Lagers 6a gleichbleibenden Wandungsdurchmesser der inneren Wandung 10 zu einer Strömungsquerschnittsverkleinerung führt. Nach Erreichen eines Maximalumfangs verringert sich der Umfang des zweiten Lagerelements 8a bzw. des sich unmittelbar anschließenden Rotorkörpers des Rotors 4. Der Strömungsquerschnitt für das von der Fluidpumpe 1a zu fördernde Fluid weist im Bereich des zweiten Lagerelements 8a des ersten mechanischen Lagers 6a somit ein lokales Strömungsquerschnittsminimum 14 auf, welches durch ein lokales Umfangsmaximum des zweiten Lagerelements 8a erreicht wird. Das lokale Strömungsquerschnittsminimum 14 ermöglicht eine Beschleunigung des Fluids über dem ersten mechanischen Lager 6a, wodurch dieses besser ausgewaschen und im Lager 6a entstehende Reibungswärme schneller abtransportiert werden kann.

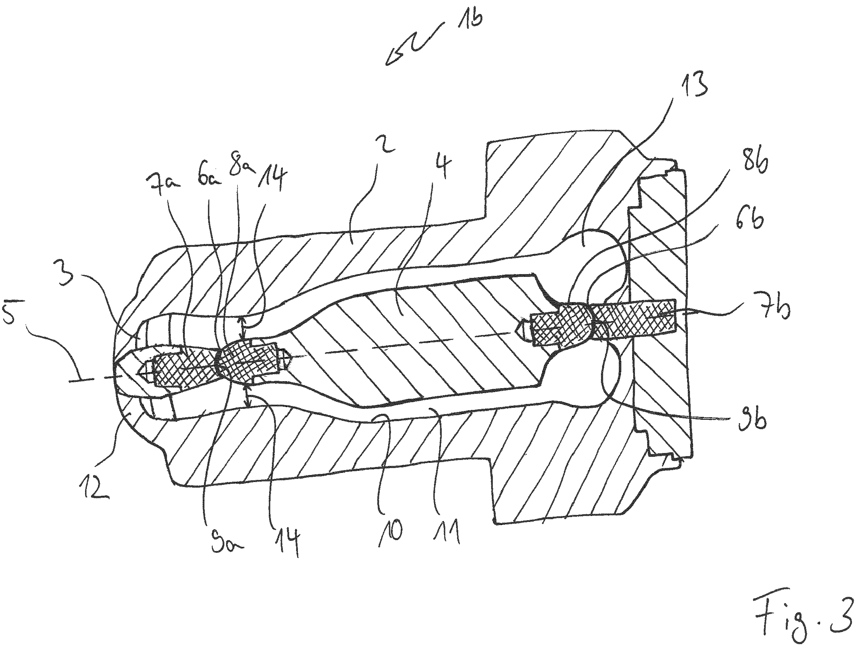

Figur 3 zeigt ein drittes Ausführungsbeispiel einer erfindungsgemäßen Fluidpumpe 1b in einer Längsschnittansicht. Die Fluidpumpe 1b ist im Wesentlichen wie die Fluidpumpe 1a des zweiten Ausführungsbeispiels ausgebildet. Die Fluidpumpe 1b unterscheidet sich von der Fluidpumpe 1a darin, dass die innere Wandung 10 im Bereich des ersten mechanischen Lagers 6a einen radialen Einzug aufweist. Somit ist der Wandungsdurchmesser der inneren Wandung 10 entlang der Rotationsachse 5 nicht konstant, sondern weist im Bereich des zweiten Lagerelements 8a des ersten mechanischen Lagers 6a ein Minimum auf. Dieses Minimum des Wandungsdurchmessers befindet sich an der Stelle entlang der Rotationsachse 5, an der der Umfang des zweiten Lagerelements 8a ein lokales Maximum aufweist. An dieser Stelle weist der Strömungsquerschnittsverlauf entlang der Rotationsachse ein lokales Strömungsquerschnittsminimum 14 auf. Durch die kombinierte Verkleinerung des Wandungsdurchmessers und Vergrößerung des Umfangs des zweiten Lagerelements 8a im Bereich des ersten mechanischen Lagers 6a kann eine stärkere Verkleinerung des Strömungsquerschnitts und somit eine größere Beschleunigung des Fluids im Bereich des ersten mechanischen Lagers 6a erreicht werden. Hierdurch kann wiederum die Auswaschung und Kühlung des ersten mechanischen Lagers 6a verbessert werden.

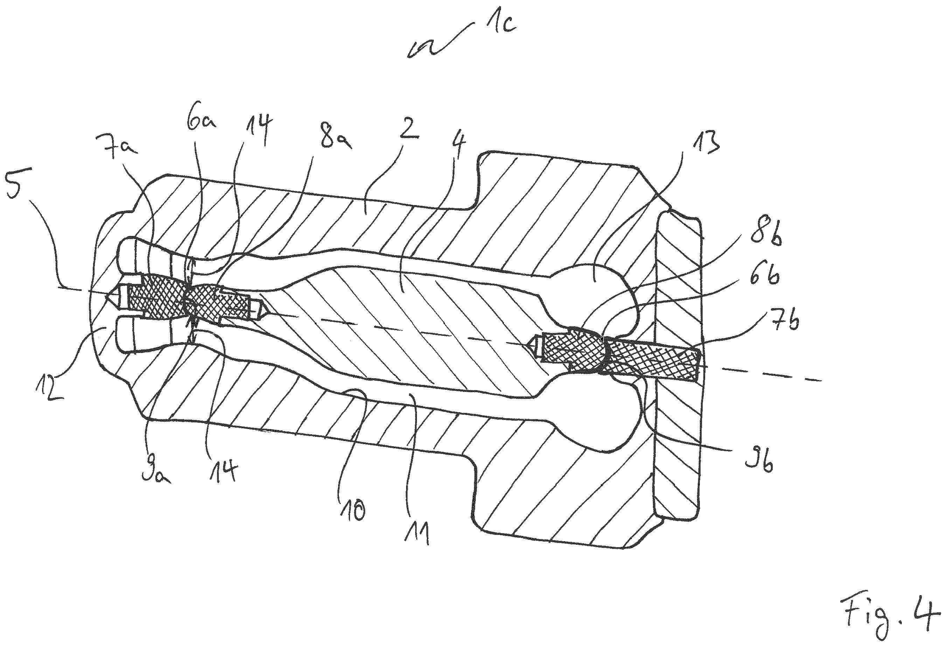

Figur 4 zeigt ein viertes Ausführungsbeispiel einer erfindungsgemäßen Fluidpumpe 1c in einer Längsschnittansicht. Die Fluidpumpe 1c der Figur 4 ist im Wesentlichen wie die Fluidpumpe 1b der Figur 3 ausgebildet. Die Fluidpumpe 1c der Figur 4 unterscheidet sich von der Fluidpumpe 1b der Figur 3 in der Position und Stärke des radialen Einzugs der inneren Wandung 10 entlang der Rotationsachse 5. Im Unterschied zur inneren Wandung 10 der Fluidpumpe 1b ist der Wandungsdurchmesser nicht im Bereich des zweiten Lagerelements 8a, sondern im Bereich des Lagerspalts 9a lokal verringert. Ferner fällt die Verringerung stärker aus als bei der inneren Wandung 10 der Fluidpumpe 1b. Somit weist die Fluidpumpe 1c im Bereich des Lagerspalts 9a des ersten mechanischen Lagers 6a ein lokales Strömungsquerschnittsminimum 14 auf.

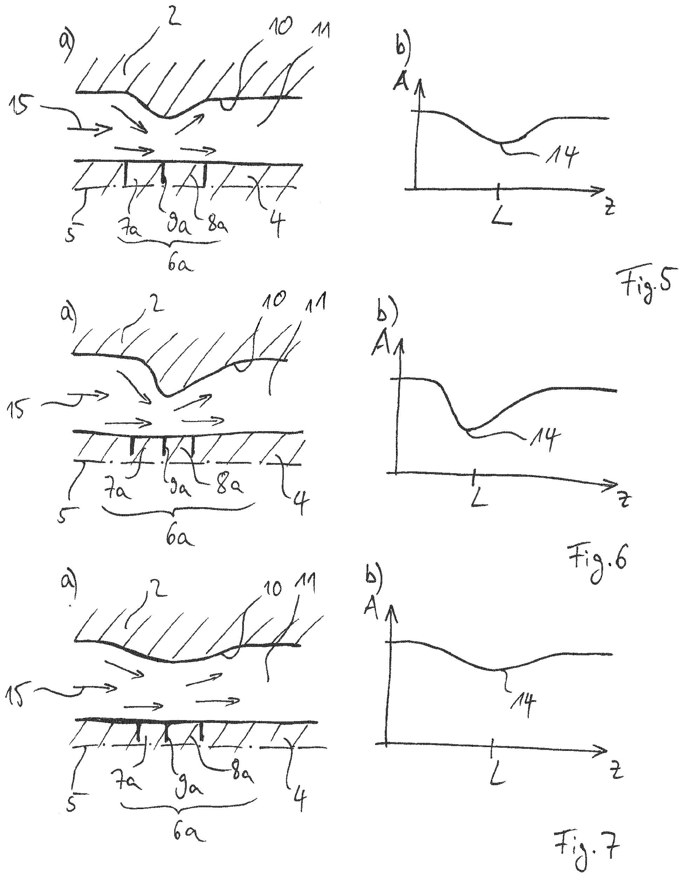

Figuren 5 bis 12 zeigen verschiedene Varianten eines erfindungsgemäßen Strömungsquerschnittsverlaufs entlang der Rotationsachse 5 im Bereich des ersten mechanischen Lagers 6a in einer schematischen Ansicht.

In Figur 5a weist die innere Wandung 10 einen radialen Einzug auf axialer Höhe des Lagerspalts 9a auf. Der radiale Einzug ist symmetrisch bezüglich des Lagerspalts 9a. Das Lager 6a selbst weist dagegen keine Aufweitung auf. Die Umfänge des ersten und zweiten Lagerelements 7a und 8a sind im Wesentlichen gleich. Daraus resultiert ein lokales Strömungsquerschnittsminimum 14 auf Höhe des Lagers 6a (L), insbesondere des Lagerspalts 9a wie in Figur 5b dargestellt ist. Figur 5b zeigt dabei den Verlauf des tatsächlichen Strömungsquerschnitts A entlang der Rotationsachse (z). Das lokale Strömungsquerschnittsminimum 14 ermöglicht eine Beschleunigung des Fluids 15 im Bereich des Lagers 6a und verbessert so eine Auswaschung und Kühlung des Lagers. Der besondere Vorteil dieser Strömungsgeometrie besteht darin, dass einfach realisierbar, richtungsunabhängig und für eine kurze Bauart, wie z.B. für kleine Fluidpumpen, geeignet ist.

Figur 6a zeigt einen bezüglich des Lagerspalts 9a asymmetrischen radialen Einzug der inneren Wandung 10, bei dem sich der Strömungsquerschnitt in Strömungsrichtung bis zum Lagerspalt 9a schnell verringert und nach durchlaufen des Strömungsquerschnittsminimums 14 langsamer wieder vergrößert. Auch in Figur 6 wird das Strömungsquerschnittsminimum allein durch einen radialen Einzug der inneren Wandung 10 ohne eine Aufweitung des ersten mechanischen Lagers 6a realisiert. Der Vorteil dieser Strömungsgeometrie besteht darin, dass die Fluidströmung stark nach innen gerichtet und über dem Lager 6a stark beschleunigt wird. Durch die langsame Vergrößerung des Strömungsquerschnitts nach Durchlaufen des Minimums wird das Ablöserisiko der Fluidströmung verringert.

Figur 7 zeigt ähnlich wie in Figur 5 ein symmetrisches Strömungsquerschnittsminimum 14, welches wieder lediglich durch einen radialen Einzug der inneren Wandung realisiert wird. Die Steigungen stromaufwärts und stromabwärts des Strömungsquerschnittsminimums 14 sind in Figur 7 jedoch geringer als in Figur 5, wodurch sich ein geringeres Ablöserisiko für die Fluidströmung ergibt. Diese Strömungsgeometrie eignet sich insbesondere für größere Fluidpumpen.

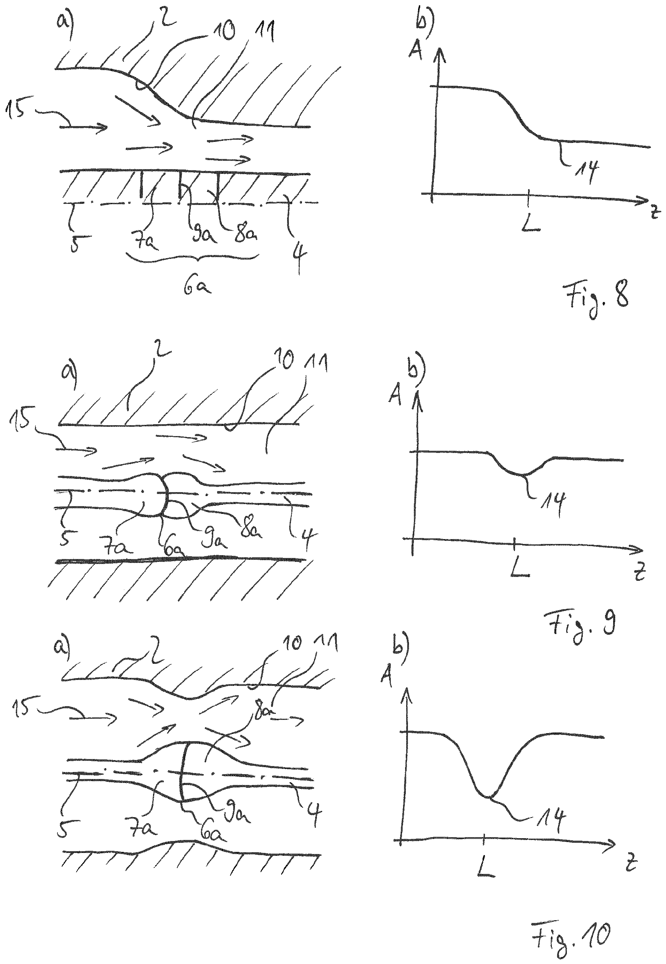

Figur 8 zeigt ein bereichsweises Strömungsquerschnittsminimum, welches durch einen bereichsweisen radialen Einzug der inneren Wandung 10 auf Höge des Lagers 6a realisiert ist. Auf Höhe des Lagerspalts 9a erreicht der Einzug ein Maximum, so dass der Wandungsdurchmesser ab dem Lagerspalt 9a bis zum Rotor 4 konstant verringert bleibt. Das bereichsweise Strömungsquerschnittsminimum 14 erstreckt sich ebenfalls in etwa vom Lagerspalt 9a bis in den Bereich des Rotors 4 hinein. Der Vorteil dieser Strömungsgeometrie besteht in der Beschleunigung des Fluidstroms über dem Lager 6a und somit in der verbesserten Auswaschung und Kühlung des Lagers 6a sowie einem geringen Ablöserisiko für die Fluidströmung ab Erreichen des Strömungsquerschnittsminimums 14.

Figur 9 zeigt ein symmetrisches Strömungsquerschnittsminimum 14 im Bereich des Lagerspalts 9a, welches nur durch eine symmetrische Aufweitung der ersten und zweiten Lagerkomponente 7a und 8a nahe des Kontaktbereichs bzw. des Lagerspalts 9a realisiert ist. Hierbei weist die innere Wandung 10 keinen radialen Einzug auf und kann entlang der Rotationsachse 5 einen konstanten Wandungsdurchmesser aufweisen. Der Vorteil dieser Strömungsgeometrie ist insbesondere, dass Gehäuse 2 (Pumpenrohr) einfach herstellbar ist. Durch die Wahl verschiedener Steigungen für die Aufweitung der Lagerkomponenten kann die Fluidströmung auf Höhe des Lagers 6a und somit eine Auswaschung und Kühlung des Lagerbereichs noch weiter optimiert werden.

Figur 10 stellt eine Kombination der Varianten aus Figur 5 und Figur 9 dar. Hierbei wird das symmetrische Strömungsquerschnittsminimum 14 sowohl durch eine symmetrische Aufweitung der Lagerkomponenten 7a und 8a als auch durch einen um den Lagerspalt 9a symmetrischen radialen Einzug der inneren Wandung 10 realisiert. Diese Kombination ermöglicht eine maximale Verkleinerung des Strömungsquerschnitts im Bereich des Lagers 6a und somit eine maximale Beschleunigung der Fluidströmung sowie verbesserte Auswaschung und Kühlung des Lagerbereichs.

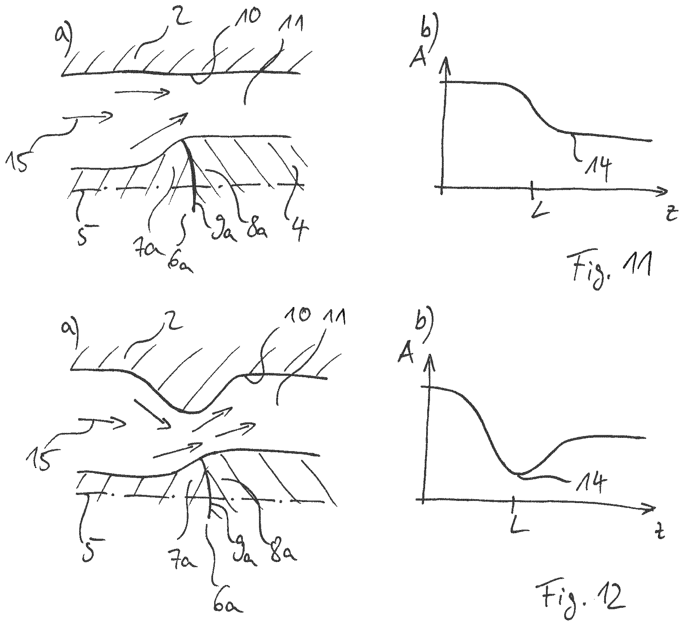

Figur 11 zeigt ein bereichsweises Strömungsquerschnittsminimum 14, welches allein durch eine Aufweitung des Lagers 6a realisiert ist. Dabei ist das erste Lagerelement 7a graduell bis zum Lagerspalt 9a aufgeweitet. Stromabwärts des Lagerspalts 9a erreicht die Aufweitung im Bereich des zweiten Lagerelements 8a ein Maximum und verbleibt maximal bis zum Bereich des Rotors 4. Der Wandungsdurchmesser der inneren Wandung 10 bleibt dabei im Bereich des Lagers 6a konstant. Entsprechend beginnt das bereichsweise Strömungsquerschnittsminimum 14 stromabwärts des Lagerspalts 9a und reicht entlang der Rotationsachse 5 bis in den Bereich des Rotors 4. Auch diese Strömungsgeometrie ermöglicht eine Beschleunigung des Fluids im Lagerbereich und dadurch eine verbesserte Auswaschung und Kühlung des Lagers 6a.

Figur 12 stellt eine Kombination der Varianten aus Figur 5 und Figur 11 dar. Figur 12 zeigt ein asymmetrisches Strömungsquerschnittsminimum 14 um den Lagerspalt 9a, welches durch eine bereichsweise Aufweitung des Lagers 6a entsprechend Figur 11 und einen symmetrischen radialen Einzug der inneren Wandung am Lagerspalt 9a realisiert ist. Stromaufwärts des Strömungsquerschnittsminimums 14 fällt der Strömungsquerschnitt in Strömungsrichtung stark ab, während der Strömungsquerschnitt stromabwärts des Strömungsquerschnittsminimums langsamer auf einen geringeren Strömungsquerschnitt als vor der Verringerung wieder ansteigt. Dies hat den Vorteil, dass einerseits eine hohe Beschleunigung des Fluids und somit gute Auswaschung und Kühlung des Lagers 6a erreicht wird, und andererseits das Ablöserisiko für die Fluidströmung an der inneren Wandung 10 nach Durchlaufen des Strömungsquerschnittsminimums 14 gering ist.

## Patentansprüche

1. Fluidpumpe zum Fördern eines Fluids, insbesondere von Blut, umfassend:
ein Gehäuse mit einem Fluideinlass und einem Fluidauslass
und einen Rotor, welcher innerhalb des Gehäuses zur Förderung des Fluids vom Fluideinlass zum Fluidauslass um eine Rotationsachse drehbar angeordnet ist,
wobei der Rotor in dem Gehäuse mittels eines mechanischen Lagers gelagert ist,
**dadurch gekennzeichnet, dass**
ein Verlauf eines Strömungsquerschnitts zwischen dem Rotor und dem Gehäuse in Richtung der Rotationsachse im Bereich des mechanischen Lagers ein lokales oder bereichsweises Strömungsquerschnittsminimum aufweist.

2. Fluidpumpe nach dem vorhergehenden Anspruch, **dadurch gekennzeichnet, dass** das mechanische Lager eine erste mit dem Gehäuse verbundene Lagerkomponente und eine zweite mit dem Rotor verbundene Lagerkomponente aufweist, wobei die erste und die zweite Lagerkomponente in Richtung der Rotationsachse in einem Kontaktbereich bis auf einen Lagerspalt aneinander anliegen, wobei sich das lokale oder bereichsweise Strömungsquerschnittsminimum im Bereich der ersten Lagerkomponente, insbesondere auf gleicher axialer Höhe wie die erste Lagerkomponente, im Bereich der zweiten Lagerkomponente, insbesondere auf gleicher axialer Höhe wie die zweite Lagerkomponente, und/oder im Bereich des Kontaktbereichs, insbesondere auf gleicher axialer Höhe wie der Kontaktbereich, befindet.

3. Fluidpumpe nach einem der vorhergehenden Ansprüche, **dadurch gekennzeichnet, dass** das Gehäuse eine einen Fluidbereich begrenzende und in einer Umfangsrichtung um die Rotationsachse geschlossene innere Wandung aufweist und das lokale oder bereichsweise Strömungsquerschnittsminimum daraus resultiert, dass ein Verlauf eines Wandungsdurchmessers der inneren Wandung in Richtung der Rotationsachse im Bereich des mechanischen Lagers ein lokales oder bereichsweises Minimum aufweist.

4. Fluidpumpe nach dem vorhergehenden Anspruch, **dadurch gekennzeichnet, dass** der Verlauf des Wandungsdurchmessers im Bereich der ersten Lagerkomponente, im Bereich der zweiten Lagerkomponente und/oder im Bereich des Kontaktbereichs ein lokales oder bereichsweises Minimum aufweist.

5. Fluidpumpe nach einem der vorhergehenden Ansprüche, **dadurch gekennzeichnet, dass** das lokale Strömungsquerschnittsminimum daraus resultiert, dass ein Verlauf eines Lagerdurchmessers des mechanischen Lagers in Richtung der Rotationsachse ein lokales oder bereichsweises Maximum aufweist.

6. Fluidpumpe nach dem vorhergehenden Anspruch, **dadurch gekennzeichnet, dass** der Verlauf des Lagerdurchmessers im Bereich der ersten Lagerkomponente, im Bereich der zweiten Lagerkomponente und/oder im Bereich des Kontaktbereichs ein lokales oder bereichsweises Maximum aufweist.

7. Fluidpumpe nach einem der vorhergehenden Ansprüche, **dadurch gekennzeichnet, dass** der Strömungsquerschnitt im lokalen oder bereichsweisen Strömungsquerschnittsminimum um ≥ 10% und/oder ≤ 50%, insbesondere ≥ 20% und/oder ≤ 40%, vorzugsweise 30% verglichen mit einem Strömungsquerschnitt in einem dem Lagerbereich stromaufwärts und/oder stromabwärts benachbarten Bereich verringert ist.

8. Fluidpumpe nach einem der vorhergehenden Ansprüche, **dadurch gekennzeichnet, dass** sich der Strömungsquerschnitt in Richtung der Rotationsachse stromaufwärts des Strömungsquerschnittsminimums um ≥ 2 % des Strömungsquerschnittsminimums pro mm und/oder ≤ 30 % des Strömungsquerschnittsminimums pro mm und stromabwärts des Strömungsquerschnittsminimums um ≤ 20 % des Strömungsquerschnittsminimums pro mm, insbesondere ≤ 10 % des Strömungsquerschnittsminimums pro mm vergrößert.

9. Fluidpumpe nach einem der vorhergehenden Ansprüche, **dadurch gekennzeichnet, dass** eine maximale Vergrößerung des Strömungsquerschnitts in Richtung der Rotationsachse stromaufwärts des lokalen oder bereichsweisen Strömungsquerschnittsminimums größer ist als oder gleich groß ist wie stromabwärts des lokalen oder bereichsweisen Strömungsquerschnittsminimums.

10. Fluidpumpe nach einem der vorhergehenden Ansprüche, **dadurch gekennzeichnet, dass** sich der Strömungsquerschnitt in Richtung der Rotationsachse stromaufwärts und/oder stromabwärts des Strömungsquerschnittsminimums innerhalb des Lagerbereichs um > 2% des Strömungsquerschnittsminimums pro mm und/oder ≤ 30 % des Strömungsquerschnittsminimums pro mm vergrößert.

11. Fluidpumpe nach einem der vorhergehenden Ansprüche, **dadurch gekennzeichnet, dass** das lokale oder bereichsweise Strömungsquerschnittsminimum ein bereichsweises Strömungsquerschnittsminimum ist und sich im Bereich des Kontaktbereichs sowie stromabwärts des Kontaktbereichs, insbesondere im Bereich des zweiten Lagerelements befindet.

12. Fluidpumpe nach einem der vorhergehenden Ansprüche, **dadurch gekennzeichnet, dass** das lokale oder bereichsweise Strömungsquerschnittsminimum derart ausgebildet ist, dass eine Wandschubspannung im Bereich des mechanischen Lagers ein lokales Maximum aufweist oder maximal ist.

13. Fluidpumpe nach einem der vorhergehenden Ansprüche, **dadurch gekennzeichnet, dass** das erste und/oder das zweite Lagerelement ein Material mit einem geringen Reibkoeffizienten und/oder mit einem hohen Wärmeleitungskoeffizienten, insbesondere Keramik, Sintermetall und/oder Diamant aufweist oder daraus besteht.
